# EUROPEAN PATENT SPECIFICATION

(11) **EP 3 870 426 B1**
(45) Date of publication and mention of the grant of the patent: **16.07.2025**
(21) Application number: 18937726.0
(22) Date of filing: 10.12.2018
(51) Int. Cl.: B29C 65/08, A61F 13/15, A61F 13/472, A61F 13/49, A61F 13/496, B29L 31/48

(54) **DUAL BONDER**
DUALER BONDER
SOUDEUSE DOUBLE

(30) Priority: 26.10.2018 US 201816172034
(43) Date of publication of application: 01.09.2021
(73) Proprietor: Curt G. Joa, Inc., Sheboygan Falls, WI 53085 (US)
(72) Inventor: SCHUETTE, David Edward, Kiel, WI 53042 (US); MCCLURG, Joram L., Port Washington, WI 53074 (US); PELLAND, Jon Allen, Sheboygan, WI 53083 (US); FRITZ, Jeffrey W., Plymouth, WI 53073 (US); PETERSON, Daniel A., Sheboygan, WI 53081 (US)
(74) Representative: Schäfer, Matthias W.
(86) International application number: PCT/US2018/064691
(87) International publication number: WO 2020/086103

(56) References cited:
- WO-A1-2013/027390
- WO-A2-2007/039755
- CN-A- 107 684 490
- US-A1- 2001 040 014
- US-A1- 2002 017 366
- US-A1- 2008 236 756
- US-A1- 2015 298 390
- US-A1- 2018 071 152
- US-A1- 2018 071 152
- US-B1- 6 454 890
- US-B2- 6 540 854

## Description

### Background of the Invention

The present invention relates to disposable hygiene products and more specifically, to apparatuses for processing disposable hygiene products such as baby diapers, adult diapers, disposable undergarments, incontinence devices, sanitary napkins and the like. More specifically, the invention relates to controlling and positioning webs or web segments of a disposable diaper and bonding them. Various types of automatic manufacturing equipment have been developed which produce the desired results with a variety of materials and configurations.

The invention disclosed herein relates to a bonding system for controlling pieces traveling on a production line, specifically a bonding system for bonding a plurality of webs together. Although the description provided relates to diaper manufacturing, the method is easily adaptable to other applications. Although the description provided relates to bonding portions of diapers, the method is easily adaptable to other products, other disposable products, other diaper types and other portions of diapers.

Such bonding systems are described in patent application CN 107 684 490 A, US 2018/071152 A1, WO 2007/039755 and WO 2013/027390 A1.

### Summary of the Invention

The present invention relates to a bonding system having the features of claim 1.

The current invention is a system for producing a disposable undergarment, with the lateral edges of the undergarment bonded by means of an ultrasonic horn. When a blank is formed for an individual undergarment, the lateral edges of opposing edges of the blank will form the seam. When the edges are sealed together, a seam is formed. The individual undergarments may be later separated along the seams.

### Brief Description of the Drawings

Figure 1 is a schematic view of a system according the present invention.
Figure 2 is a view similar to that of Figure 1, but showing movement of the component parts to vary the distance between bonds.
Figure 3 is an enlarged view of an anvil insert for use with the present system.
Figure 3A is an enlarged perspective view of the anvil insert illustrated in Figure 3.
Figure 4 is a top view of the anvil insert illustrated in Figure 3.
Figure 4A is an enlarged view of Figure 4 and showing a bond pattern.
Figure 4B is a cross sectional view of the anvil insert illustrated in Figure 4A, and taken along lines 4B - 4B thereof.
Figure 4C is an enlarged view of Figure 4A.
Figure 5 is a top plan view showing a web with spaced apart bonds.
Figure 6 is a schematic view similar to that of Figure 1, and showing webs moving at a non-bonding velocity.
Figure 6A is a view similar to that of Figure 6, but showing a front side and a back side of a folded web.
Figure 6B is a cross sectional view taken along lines 6B -6B of Figure 6A, and showing a back side adjacent an anvil and a front side adjacent a horn.
Figure 7 is a schematic view similar to that of Figure 6, and showing movement of accumulator rollers to slow the web to a bonding velocity.
Figure 8 is a schematic view similar to that of Figures 6 and 7, and showing movement of accumulator rollers to increase the velocity web after bonding.
Figure 9 is a top plan view of webs moving as illustrated in Figure 6.
Figure 10 is a top plan view of webs moving as illustrated in Figure 7.
Figure 11 is a graphic representation of the various web velocities.
Figure 12 is a view similar to that of Figure 1, but showing three anvils and ultrasonic horns.
Figure 13 is a view similar to that of Figure 12, but showing movement of the component parts to vary the distance between bonds.
Figure 14 is a top plan view showing paired anvils and ultrasonic horns.
Figure 15 is a view similar to that of Figure 14, but showing movement of the component parts to vary the distance between bonds.
Figure 16 is a view similar to that of Figures 1, 6, and 7 but showing an accumulator series.
Figure 17 is a view similar to that of Figures 1, 6, 7 and 16 but showing another alternative arrangement for the accumulator series.
Figure 18 is a view similar to that of Figures 1, 6, 7, 16 and 17 but showing another alternative arrangement for the accumulator series.
Figure 19 is a view similar to that of Figures 1, 6, 7, 16, 17 and 18 but showing another alternative arrangement for the accumulator series.
Figure 20 is a view similar to that of Figures 1, 6, 7, 16, 17, 18 and 19 but showing servo motor driven anvils.
Figure 21 is a top plan view of the arrangement illustrated in Figure 20 and showing paired servo driven anvils.
Figure 22 is an end view of a servo driven anvil and showing various rotational velocity employed during use.
Figure 23 is an enlarged view of an anvil insert for use with the present system, similar to that of Figure 3, but showing another seal and emboss surface arrangement.
Figure 24 is a top view of the anvil insert illustrated in Figure 23.
Figure 24A is an enlarged view of Figure 24 and showing a bond pattern.
Figure 25 is a top view of another anvil according to the present invention in which the surface of the anvil includes a seal and emboss surface arrangement.
Figure 26 is a top view of another anvil according to the present invention and having a curved anvil insert.
Figure 26A is an enlarged view of Figure 26 and showing a bond pattern.
Figure 27 is a top plan view of webs moving and use of the anvil illustrated in Figure 26.
Figure 28 is a top view of another anvil similar to that of Figure 25, but showing another seal and emboss surface arrangement in which the bond pattern is arranged in a sinusoidal configuration.

### Description of the Preferred Embodiment

Although the disclosure hereof is detailed and exact to enable those skilled in the art to practice the invention, the physical embodiments herein disclosed merely exemplify the invention which may be embodied in other specific structures. While the preferred embodiment has been described, the details may be changed without departing from the invention.

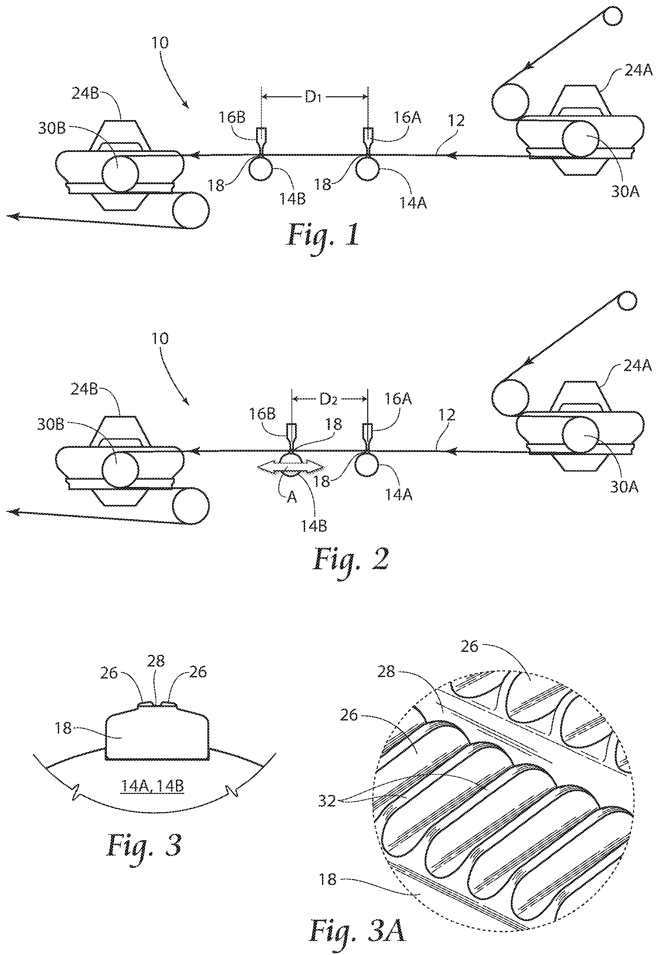

With attention to Figures 1 and 2, a bonding system 10 is disclosed wherein the positional accuracy of the bonding and quality of the bonding is improved over the prior art. As shown, the system 10 includes an ultrasonic bonder for bonding a plurality webs 12, or a folded web 12 having a front side 12A and a back side 12B. The system 10 includes a first anvil 14A, a second anvil 14B, a first ultrasonic horn 16A, and a second ultrasonic horn 16B. The anvils 14A, 14B are each provided with an anvil insert 18 having a predetermined profile. The first and second anvils 14A, 14B are laterally spaced apart inline and in a machine direction.

The system 10 includes carrying means for carrying the webs 12 so that the webs 12 pass a first gap between the first anvil 14A and the first ultrasonic horn 16A and then a second gap between the second anvil 14B and the second ultrasonic horn 16B. The first and second ultrasonic horns 16A, 16B apply vibration energy to the web 12 simultaneously and in cooperation with a respective anvil 14A, 14B to bond a respective portion of the web 12 that is to be an end portion 20 of an individual finished article (not shown in these views). As mentioned, the first anvil 14A and the first ultrasonic horn 16A are provided inline from the second anvil 14B and the second ultrasonic horn 16B and are spaced apart a predetermined distance *d1* that corresponds to the distance between bonds 22. The predetermined distance *d1* may be changed to accommodate various sizes of the finished product, since the distance *d1* corresponds to the length of the individual article. Accordingly, the length of the individual article may be changed by adjusting the position of the first or second ultrasonic horn 16A, 16B with respect to the other ultrasonic horn 16A, 16B. Moreover, the system 10 includes a device for linear reciprocation of a selected anvil 14A, 14B and ultrasonic horn 16A, 16B relative another anvil 14A, 14B and ultrasonic horn 16A, 16B and to move in the direction of arrow A, (see Figure 2) and to thereby change the distance *dl, d2* between a selected anvil 14A, 14B and horn 16A, 16B and the adjacent anvil 14A, 14B and horn 16A, 16B. Preferably, the selected anvil 14A, 14B and ultrasonic horn is 16A, 16B slidingly mounted to a base structure (not shown), and its movement manually or computer controlled, as understood by one skilled in the art. Since the distance *dl, d2* intervals of bonding positions may be changed by changing the position of the first or second ultrasonic horn 16A, 16B, the present system may easily produce individual articles of various sizes. It is to be understood that while the view of Figure 2 illustrates movement of the second ultrasonic horn 16B, the position of the first ultrasonic horn 16A may also or alternatively be changeable, as required by a specific application.

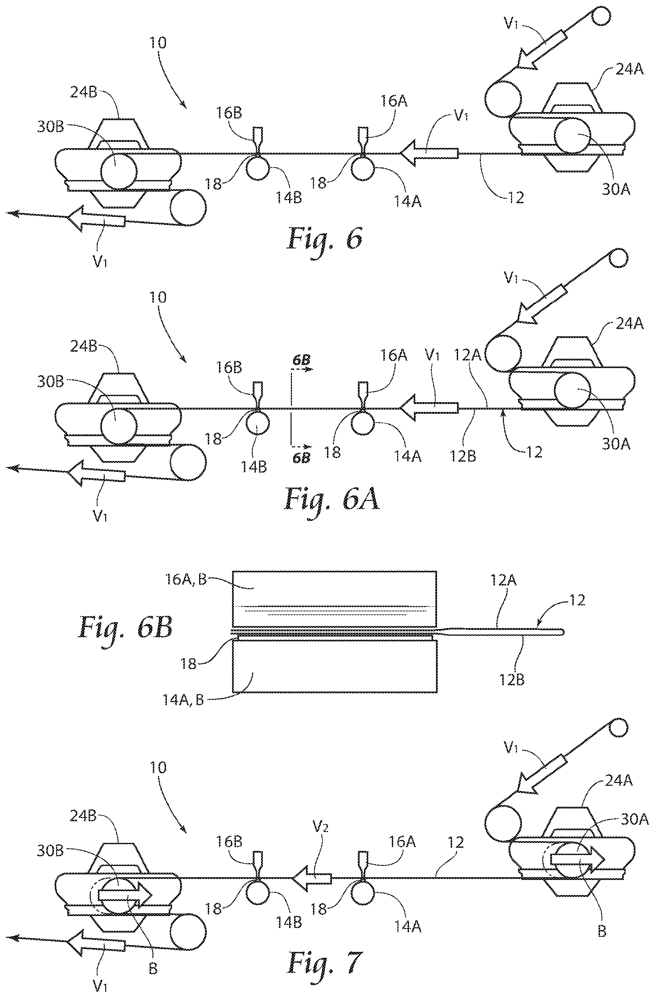

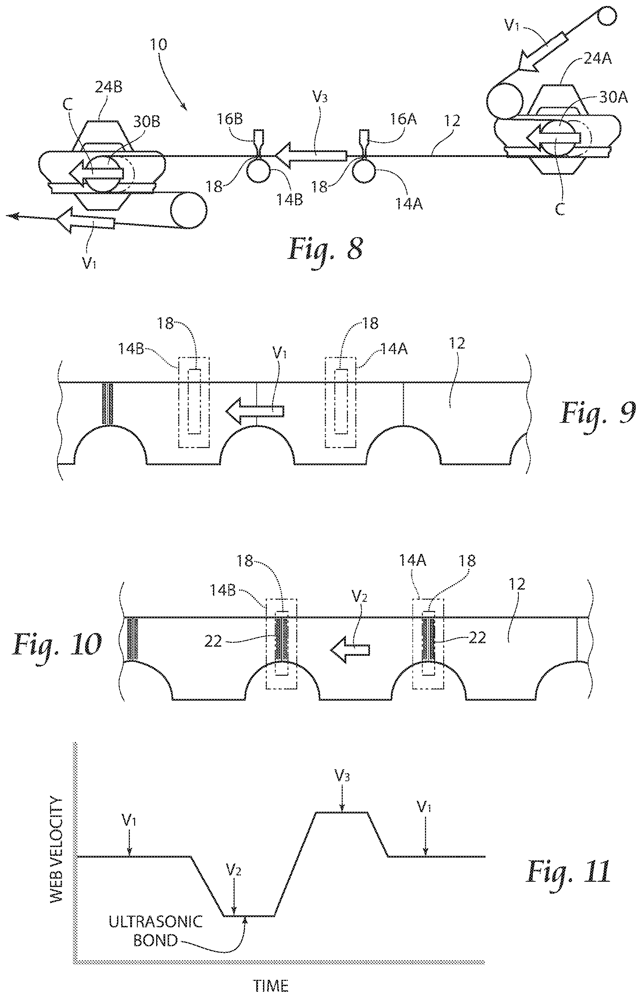

With attention to Figures 6 - 11, the present system 10 may be seen to further comprise a velocity-changing device for increasing and decreasing the moving velocity of the web 12. The velocity-changing device preferably includes a first web festoon accumulator 24A having a first accumulator roller 30A, and a second web festoon accumulator 24B having a second accumulator roller 30B. The first web festoon accumulator 24A receives the webs 12 flowing from an upstream side and releases the webs toward the ultrasonic horns 16A, 16B while the second web festoon accumulator 24B receives the webs 12 from the ultrasonic horns 16A, 16B and moves the webs 12 toward a downstream side. The velocity-changing device further includes means for moving the first and second accumulator rollers 30A, 30B in a unison, linear manner to thereby change the velocity V1 of the web 12 received. As seen in Figure 7, when the first and second accumulator rollers 30A, 30B move in the direction of arrow B, the velocity V1 of the web 12 from the upstream side is moved to second, slower velocity V2, such that the dwell time of the web 12 during the bonding operation is adequate for proper bonding. The anvil rolls 14A, 14B are preferably synchronized such that the device 10 will produce two bonds 22 simultaneously during the slower V2 velocity. Once the web 12 is bonded, the accumulator rollers 30A, 30B move in the direction of arrow C (see Figure 8) and the webs 12 move at velocity V3 to be ultimately transported by the second web festoon accumulator 24B at the first V1 velocity and in a downstream direction.

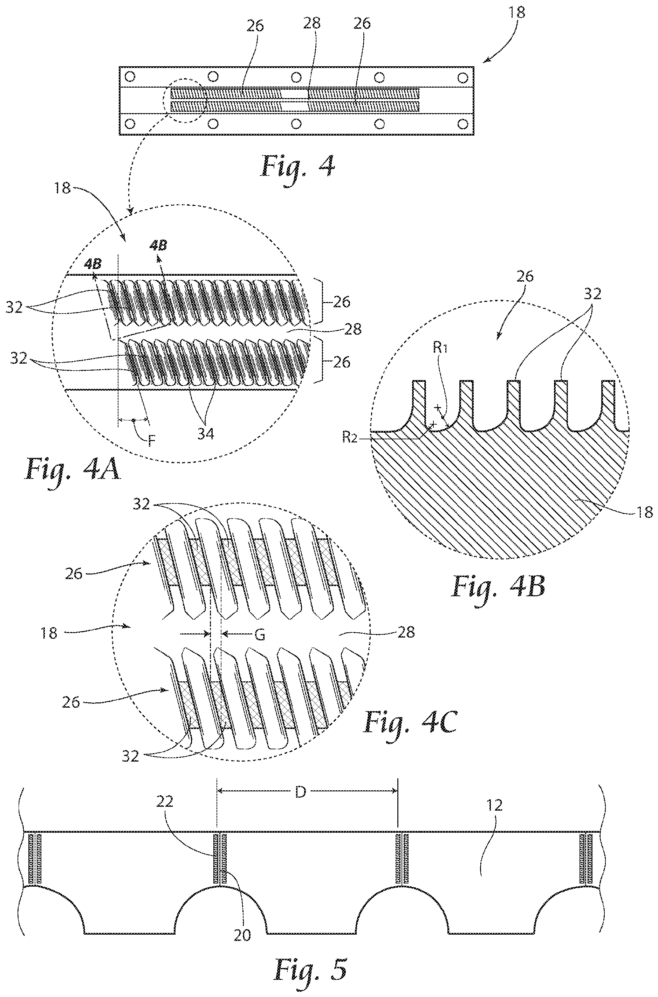

The surfaces of the anvils 14A, 14B used in cooperation with the ultrasonic horns 16A, 16B may preferably include an anvil insert 18, as is shown in Figures 3 -4C. The anvil insert 18 may include a seal surface, an embossing surface, or a combination thereof. The anvil insert 18 illustrated in these views includes a pair of spaced apart seal surfaces 26 having a recess 28 therebetween and wherein the seal surfaces 26 are provided with a series of canted rectangular patterns or teeth 32 thereon. The canted orientation of the rectangular pattern 32 provides both trailing edge and leading edge coverage in a cross-machine direction. This arrangement allows even wear on the horns 16A, 16B interfacing with the anvils 14A, 14B such that the need to re-grind worn horns 16A, 16B is greatly reduced. Moreover, the cost associated with anvil 14A, 14B assembly is reduced because the anvil 14A, 14B and the insert 18 may be manufactured separately and less material is required. Further, maintenance of the anvil 14A, 14B is easier and less costly since the user requires only a spare insert 18 rather than an entire anvil 14A, 14B when replacement is needed. Typical bond patterns produced by typical anvils (not shown) are not canted and are often merely a series of parallel rectangles (not shown). During use, these typical bond patterns may wear grooves into the surface of the horn 16A, 16B causing downtime for horn 16A, 16B maintenance. The pattern 32 disclosed in Figures 4, 4A, 4B and 4C reduces downtime for horn 16A, 16B maintenance. With particular reference to Figures 4A and 4B, it may be seen that the canted arrangement of the rectangles or teeth 32, creates a bond pattern that will evenly wear a corresponding ultrasonic horn 16A, 16B. As shown, the edges 34 of adjacent teeth 32 are parallel to one another for facile manufacture. Moreover, the teeth 32 are angled relative to the machine direction at a predetermined angle F (see Figure 4A) that provides a following tooth 32 to fill in any gaps G (See Figure 4C) existing between any preceding tooth 32. The view of Figure 4C illustrates this particular feature in greater detail. Depending on the geometry, such as width of gap between teeth 32 rows, width of gap between teeth 32, and the like, the predetermined angle F may provide full coverage of the ultrasonic horn 16A, 16B to achieve the goal of even wear. While angling the teeth 32 provides more even wear of the ultrasonic horn 16A, 16B, structural liability of the teeth 32 may increase with the angle F. Structural integrity of the teeth 32 may be increased through the use of various radii between the teeth 32 (see Figure 4B for example). By varying the radii between the teeth 32 such that each tooth has a small radius R2 on one side and a large radius R1 on the other, individual teeth 32 are more structurally sound and the chance of breaking a tooth 32 is greatly reduced. The anvil insert 18 of these views may be used to simultaneously bond adjacent article end portions 20, while reserving a boundary between the sealed end portions for a later severing operation.

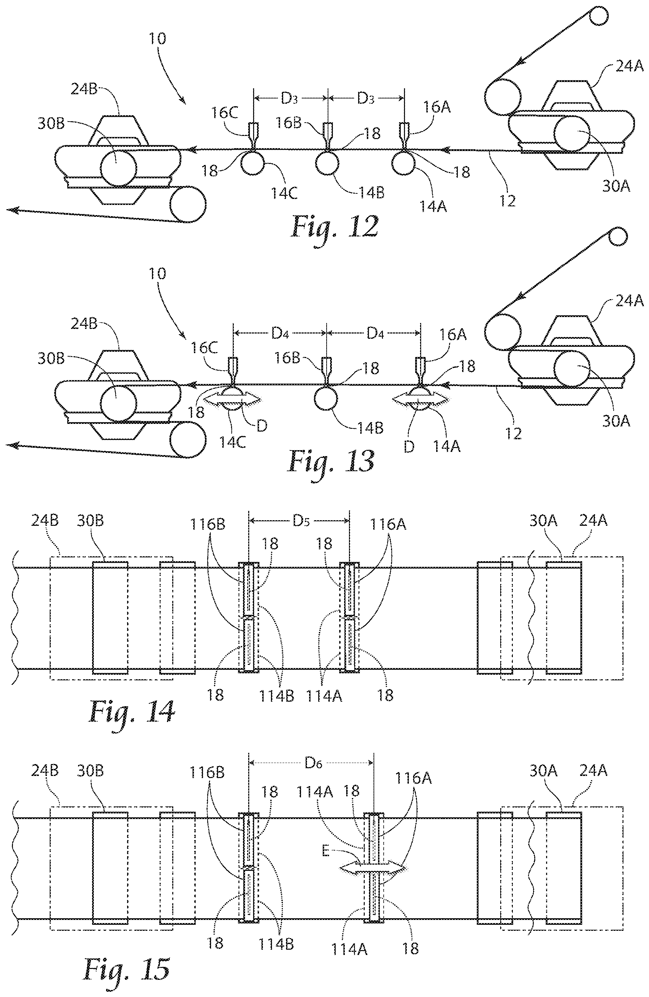

With attention to Figures 12 - 15, it may be seen that a bonding system 10 according to the present invention may include a first anvil 14A, a second anvil 14B, a third anvil 14C, a first ultrasonic horn 16A, a second ultrasonic horn 16B, and a third ultrasonic horn 16C. The anvils 14A, 14B, 14C may be each provided with an anvil insert 18 having a predetermined profile, as described above. The anvils 14A, 14B, 14C are laterally spaced apart inline and in a machine direction. As in the previous embodiment, the ultrasonic horns 16A, 16B, 16C apply vibration energy to the web 12 simultaneously and in cooperation with a respective anvil 14A, 14B, 14C to bond a respective portion of the web 12 that is to be an end portion 20 of an individual finished article (not shown in these views). As previously described, the first anvil 14A and the first ultrasonic horn 16A are provided inline from the second anvil 14B and the second ultrasonic horn 16B, with the third anvil 14C and the third ultrasonic horn 16C provided inline from the second anvil 14B and the second ultrasonic horn 16B. The anvils 14A, 14B, 14C with the corresponding horns 16A, 16B, 16C are spaced apart a predetermined distance d3 that corresponds to the distance between bonds 22. The predetermined distance *d3* may be changed to accommodate various sizes of the finished product, since the distance *d3* corresponds to the length of the individual article. Accordingly, the length of the individual article may be changed by adjusting the position of the first, second, or third ultrasonic horn 16A, 16B, 16C with respect to any other ultrasonic horn 6A, 16B, 16C. Moreover, the system 10 includes a device for linear reciprocation of a selected anvil 14A, 14B, 14C and ultrasonic horn 16A, 16B, 16C relative to another anvil 14A, 14B, 14C and ultrasonic horn 16A, 16B, 16C and to move in the direction of arrow D, (see Figure 13) and to thereby change the distance *d3, d4* between a selected anvil 14A, 14B, 14C and horn 16A, 16B, 16C and the adjacent anvil 114A, 14B, 14C and horn 16A, 16B, 16C. As in the previously described arrangement, a selected anvil 14A, 14B, 14C and ultrasonic horn is 16A, 16B, 16C is preferably slidingly mounted to a base structure (not shown), and the movement manually or computer controlled, as understood by one skilled in the art. Since the distance *d3, d4* intervals of bonding positions may be changed by changing the position of the first, second, or third ultrasonic horn 16A, 16B, 16C the present system may easily produce individual articles of various sizes. It is to be understood that while the views of Figures 12 and 13 illustrate movement of the first and third ultrasonic horns 16A, 16C, the position of the second ultrasonic horn 16B may also or alternatively be changeable, as required by a specific application. Moreover, the bonding system 10 may include any combination of fixed and moveable ultrasonic horns 16A, 16B, 16C, such as, but not limited to: one fixed ultrasonic horn, with two movable ultrasonic horns; two fixed ultrasonic horns and one movable ultrasonic horn; three fixed ultrasonic horns; and three movable ultrasonic horns, by way of non-limiting example.

Figures 14 and 15 illustrate another arrangement of anvils and ultrasonic horns. In these views anvil pairs 114A, 114B are utilized rather that the single anvils 14A, 14B, 14C illustrated in previous views. Moreover, ultrasonic horn pairs 116A, 116B correspond to and cooperate with the anvil pairs 114A, 114B.

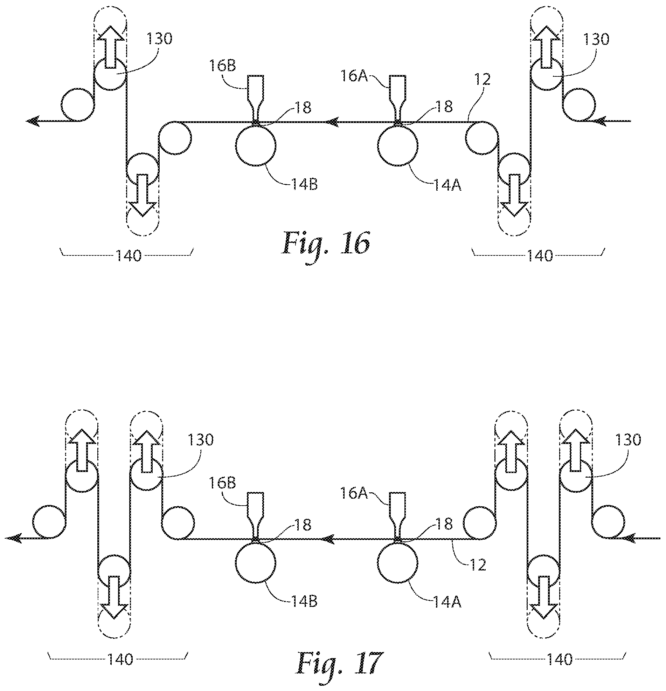

Figures 16 and 17 illustrate an alternative arrangement and showing a vertical accumulator series 140 rather than the web festoon accumulator 24A, 24B and accumulator rollers 30A, 30B arrangement previously described. As shown, the accumulator series 140 may include any number of roll assemblies 130. It is to be understood that while specific numbers and arrangements of assemblies 130 are shown in the Figures, any number and arrangement of roll assemblies 130 may be envisioned without departing from the invention. Moreover, while not specifically shown, it is to be understood that the accumulator series 140 shown in Figures 16 and 17 may be used with any of the anvils 14A, 14B, 14C and ultrasonic horns 16A, 16B, 16C described and illustrated in previous views.

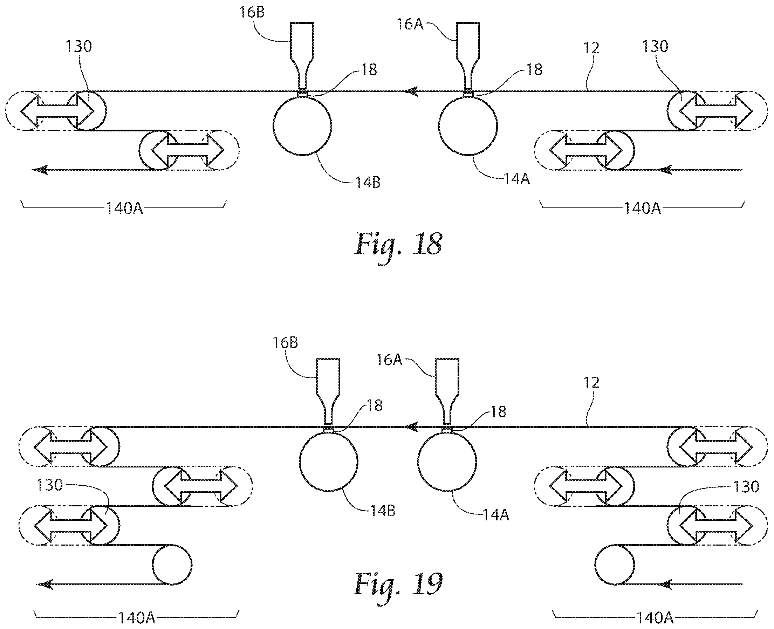

Figures 18 and 19 illustrate an alternative arrangement similar to that of Figures 16 and 17 but showing a horizontal accumulator series 140A. As shown, the accumulator series 140A may include any number of roll assemblies 130. As in the arrangements shown n Figures 16 and 17, it is to be understood that the specific number and arrangement of assemblies 130 shown in the Figures should not be considered limiting, and any number and arrangement of roll assemblies 130 may be envisioned without departing from the invention. Moreover, while not specifically shown, it is to be understood that accumulator series 140A shown in Figures 18 and 19 may be used with any of the anvils 14A, 14B, 14C and ultrasonic horns 16A, 16B, 16C described and illustrated in previous views.

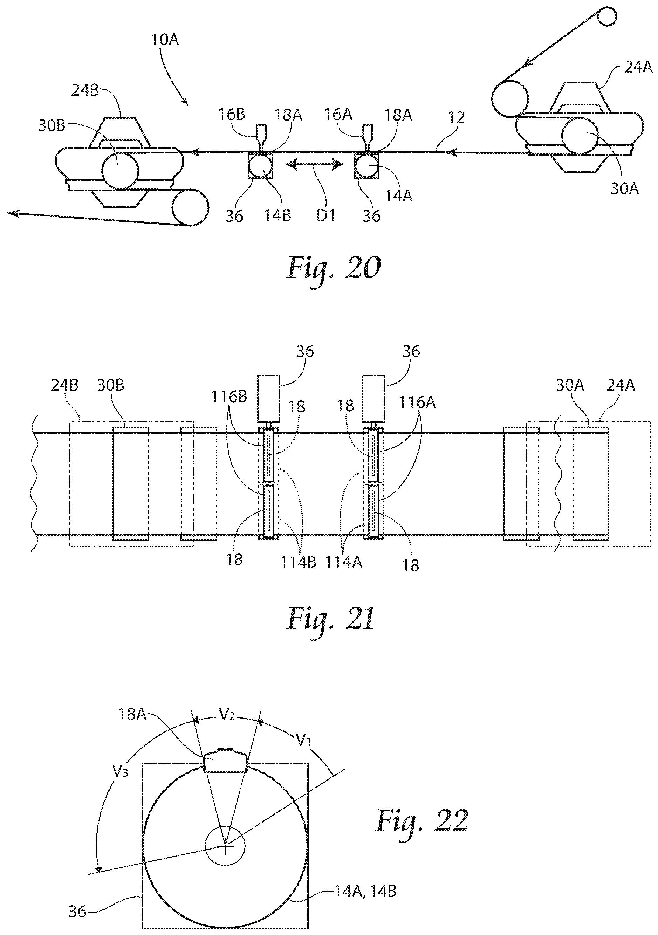

With attention to Figures 20 and 21, another bonding system 10A may be seen. As shown, and similar to the system described with reference to Figures 1 and 2, system 10 includes an ultrasonic bonder for bonding a plurality webs 12, or a folded web 12. As in the previously described system, the system 10A shown in these views includes a first anvil 14A, a second anvil 14B, a first ultrasonic horn 16A, and a second ultrasonic horn 16B. The anvils 14A, 14B are each provided with an anvil insert 18A having a predetermined profile. The first and second anvils 14A, 14B are laterally spaced apart inline and in a machine direction. The anvils 14A, 14B seen in views are each further provided with a servo motor 36 such that the anvils 14A, 14B may be servo motor driven. As seen in Figure 22, the servo motor 36 functions to vary the revolution speed of the anvils 14A, 14B. The speed may be varied to thereby influence the dwell time of the anvil insert 18A against the web 12. For example, and as shown in Figure 22, the revolution speed of the anvils 14A, 14B is varied such that, the revolution speed V1 of the anvils 14A, 14B from the upstream side may be slowed to second, slower velocity V2, such that the dwell time of the web 12 during the bonding operation is adequate for proper bonding. The anvils 14A, 14B are preferably synchronized such that the device 10A will produce two bonds 22 simultaneously during the slower V2 velocity. Once the web 12 is bonded, the anvils 14A, 14B accelerate to velocity V3 to be rotated back to the first V1 velocity, and in a downstream direction.

As in the previous embodiment, the system 10A shown in these views includes carrying means for carrying the webs 12 so that the webs 12 pass a first gap between the first anvil 14A and the first ultrasonic horn 16A and then a second gap between the second anvil 14B and the second ultrasonic horn 16B. The first and second ultrasonic horns 16A, 16B apply vibration energy to the web 12 simultaneously and in cooperation with a respective anvil 14A, 14B to bond a respective portion of the web 12 that is to be an end portion 20 (see Figure 5) of an individual finished article (not shown in these views). As mentioned, the first anvil 14A and the first ultrasonic horn 16A are provided inline from the second anvil 14B and the second ultrasonic horn 16B and are spaced apart a predetermined distance *d1* that corresponds to the distance between bonds 22. The predetermined distance *d1* may be changed to accommodate various sizes of the finished product, since the distance *d1* corresponds to the length of the individual article. Accordingly, the length of the individual article may be changed by adjusting the position of the first or second ultrasonic horn 16A, 16B with respect to the other ultrasonic horn 16A, 16B.

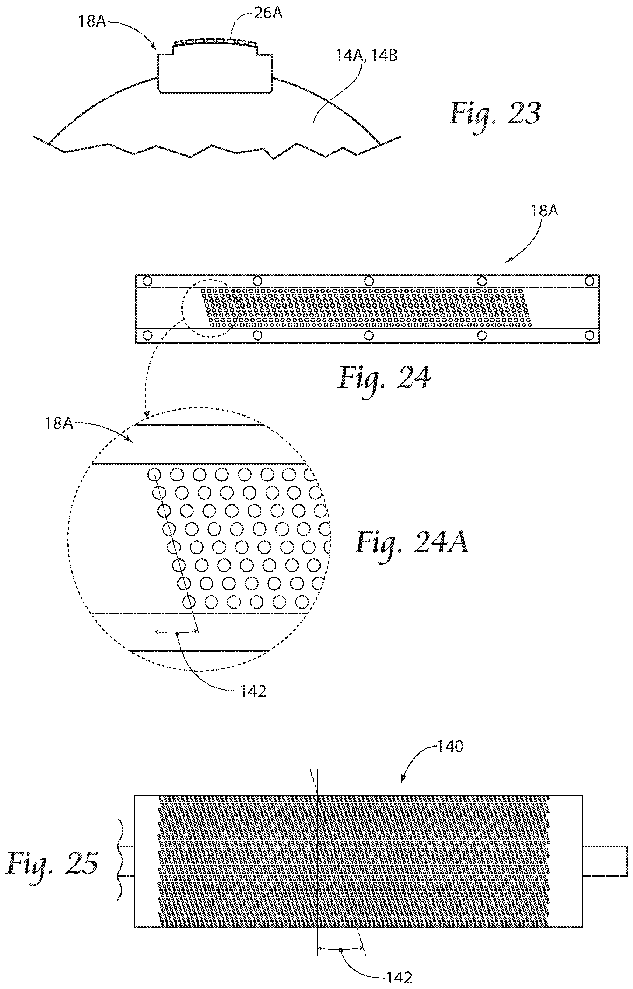

As in the previous embodiment, the surfaces of the anvils 14A, 14B used in cooperation with the ultrasonic horns 16A, 16B may include an anvil insert 18A, as is shown in Figures 23 -24A. The anvil insert 18A may include a seal surface, an embossing surface, or a combination thereof. The anvil insert 18A illustrated in these views includes a seal surface having a plurality of raised seal areas 26A and wherein the seal areas 26A are arranged in a pattern that is canted from the machine direction in an angle 142 (see Figures 24, 24A). Alternatively, and as seen in Figure 25, the pattern shown in Figures 24, 24A may be applied to the entire anvil 140.

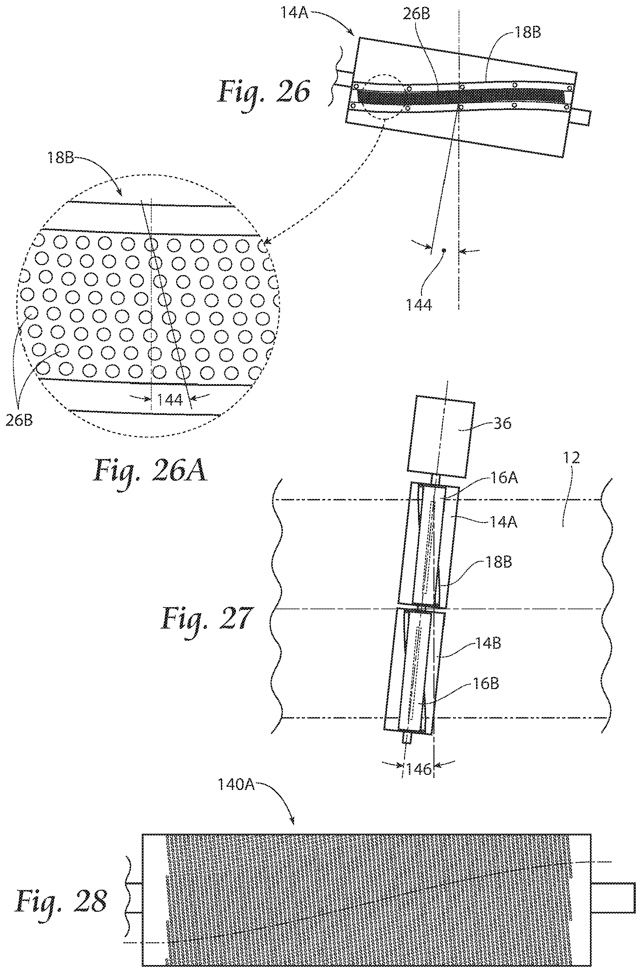

With reference to Figures 26 - 27, it may be seen that the surfaces of the anvils 14A, 14B may include another anvil insert 18B. As shown, and similar to the views of Figures 23 - 24A, the seal surface of these views includes a plurality of raised seal areas 26B which are arranged in a pattern having an angle 144 (see Figure 26A) relative to the anvil 14A, 14B. The anvil insert 18B is further curved in a helical configuration. As seen in Figure 27, the angle 144 of the seal area 26B pattern shown in Figure 26A may be applied (inversely) to the entire unit (horn 16A, 16B and servo-driven anvil roll 14A, 14B) such that the angled seal surface 26B pattern is parallel to the machine direction when the horn 16A, 16B and servo-driven anvil roll 14A, 14B are canted in the cross direction by the inverse angle 146. Alternatively, and as seen in Figure 28, the seal area 26B pattern shown in Figures 25 - 27 may be applied to the surface of the entire anvil 140A in a helical pattern. As in the arrangement of Figures 26 - 27, the seal surface 26B pattern of anvil 140A is also parallel to the machine direction when the horn 16A, 16B and servo-driven anvil roll 140A is canted in the cross direction by the inverse angle 146 (see Figure 27). By canting the horn 16A, 16B and servo-driven anvil roll 140A in the angle 146 seen in Figure 27 the pattern on the anvil 140A is applied in a parallel fashion of the web 12.

The arrangements shown in the views of Figures 26 - 28 increase the anvil 14A, 140A dwell time against the web 12 during bond formation and optimizes the force and power of the bond action. The angle 146 of the horn 16A, 16B and anvil 14A, 140A relative to the angle 144 of the seal surface 26B pattern increases the web 12 leading edge bond. The arrangement increases the typical time that the anvil 140A interacts with the horn 16A, 16B. Moreover, the arrangement minimizes certain known issues with consistent intermittent bond strength, such as spikes in power and weak leading edge bonds. The anvil and horn angle 146 in relation to the running web 12, along with the helical pattern of the seal surface 26B pattern produce a bond that is perpendicular to the machine direction. The arrangement of the helical pattern on the anvil insert 26B and anvil 140A, along with the canted orientation of the anvil 14A, 14B, 140A and horn 16A, 16B relative the machine direction results in less area under the horn 16A, 16B at any point in the bond thereby allowing the potential for higher bond forces. The anvil 14A, 14B, 140A and horn 16A, 16B canted relative the machine direction may preferably be in the range of 5 - 15°

The angled orientation of the seal surface 26B pattern improves bond coverage in a cross-machine direction and further allows even wear on the horns 16A, 16B interfacing with the anvils 14A, 14B, 140 such that the need to re-grind worn horns 16A, 16B is greatly reduced. As mentioned, typical bond patterns produced by typical anvils (not shown) are not canted and are often merely a series of parallel rectangles (not shown). During use, these typical bond patterns may wear grooves into the surface of the horn 16A, 16B causing downtime for horn 16A, 16B maintenance. The patterns 32 disclosed in Figures 24, 25, 26 and 28 reduce downtime for horn 16A, 16B maintenance. The anvil insert 18 in the views of Figures 24 and 26 may be used to simultaneously bond adjacent article end portions 20, while reserving a boundary between the sealed end portions for a later severing operation.

It is to be understood that the anvils 14A, 14B, 140A, 140B described above may be utilized singly or in pairs as illustrated, for example in Figure 27. Moreover, ultrasonic horns 16A, 16B may be paired correspond to and cooperate with paired anvils 14A, 14B, 140A, 140B.

The foregoing is considered as illustrative only of the principles of the invention. Furthermore, since numerous modifications and changes will readily occur to those skilled in the art, it is not desired to limit the invention to the exact construction and operation shown and described. While the preferred embodiment has been described, the details may be changed without departing from the invention, which is defined by the claims.

## Claims

1. A bonding system (10) for bonding webs including:
a first anvil (14A) and a corresponding first ultrasonic horn (16A);
a second anvil (14B) and a corresponding second ultrasonic horn (16B), wherein each of the first anvil (14A) and the second anvil (14B) includes a seal surface (26B) having a predetermined profile;
wherein said first anvil (14A) and the second anvil (14B) are laterally spaced apart inline and in a machine direction;
wherein said first anvil (14A) and said second anvil (14B) are each provided with a respective servo motor;
**characterised in that**
said seal surface of each of the first anvil and the second anvil comprises an anvil insert having a predetermined seal surface pattern;
and **in that** said seal surface pattern is a helical pattern.

2. The system of claim 1 wherein said seal surface (26B) of each of the first anvil (14A) and the second anvil (14B) is arranged in a curved helical pattern.

3. The system of claim 1 or 2 wherein said seal surface (26B) of each of the first anvil (14A) and the second anvil (14B) includes a plurality of raised seal areas which are arranged in a pattern having an angle (144) relative to the anvil (14A, 14B).

4. The system of any of claim 1 to 3 wherein an anvil and horn angle (146) in relation to the running web (12), along with the helical pattern of the seal surface (26B) is configured to produce a bond that is perpendicular to a machine direction.

5. The system of any of claim 1 to 4 wherein the anvil (14A, 14B) has a canted orientation.

6. Disposable hygiene product produced with a bonding system (10) of any of claim 1 to 5.

## Patentansprüche

1. Bondingsystem (10) zum Bonden von Vlies, einschließlich:
einen ersten Amboss (14A) und ein entsprechendes erstes Ultraschallhorn (16A);
einen zweiten Amboss (14B) und ein entsprechendes zweites Ultraschallhorn (16B), wobei jeder der ersten Ambosse (14A) und der zweiten Ambosse (14B) eine Dichtungsfläche (26B) mit einem vorgegebenen Profil aufweist;
wobei der erste Amboss (14A) und der zweite Amboss (14B) seitlich in Reihe und in einer Maschinenrichtung voneinander beabstandet sind;
wobei der erste Amboss (14A) und der zweite Amboss (14B) jeweils mit einem jeweiligen Servomotor versehen sind;
**dadurch gekennzeichnet, dass**
die Dichtungsfläche jedes der ersten und zweiten Ambosse einen Ambosseinsatz mit einem vorgegebenen Dichtungsoberflächenmuster umfasst; und dass
das Dichtungsoberflächenmuster ein Helixmuster ist.

2. System nach Anspruch 1, wobei die Dichtungsfläche (26B) jeweils des ersten Ambosses (14A) und des zweiten Ambosses (14B) in einem gekrümmten Helixmuster angeordnet ist.

3. System nach Anspruch 1 oder 2, wobei die Dichtungsfläche (26B) jedes der ersten Ambosse (14A) und der zweiten Ambosse (14B) eine Vielzahl von erhabenen Dichtungsbereichen umfasst, die in einem Muster angeordnet sind, das einen Winkel (144) relativ zum Amboss (14A, 14B) aufweist.

4. System nach einem der Ansprüche 1 bis 3, wobei ein Amboss- und Hornwinkel (146) in Bezug auf die Laufrichtung des Vlies (12) zusammen mit dem Helixmuster der Dichtungsfläche (26B) so ausgebildet ist, dass eine Bindung erzeugt wird, die senkrecht zu einer Maschinenrichtung verläuft.

5. System nach einem der Ansprüche 1 bis 4, wobei der Amboss (14A, 14B) eine geneigte Ausrichtung aufweist.

6. Einweg-Hygieneprodukt, hergestellt mit einem Bondingsystem (10) nach einem der Ansprüche 1 bis 5.

## Revendications

1. Système de soudage (10) destiné à soudager des bandes incluant :
une première enclume (14A) et un premier avertisseur à ultrasons correspondant (16A) ;
une seconde enclume (14B) et un second avertisseur à ultrasons correspondant (16B), dans lequel chacune de la première enclume (14A) et de la seconde enclume (14B) inclut une surface d'étanchéité (26B) présentant un profil prédéterminé ;
dans lequel ladite première enclume (14A) et la seconde enclume (14B) sont espacées latéralement en ligne et dans une direction de la machine ;
dans lequel ladite première enclume (14A) et ladite seconde enclume (14B) sont chacune munies d'un servomoteur respectif ;
**caractérisé en ce que**
ladite surface d'étanchéité de chacune de la première enclume et de la seconde enclume comprend une pièce d'insertion d'enclume présentant un motif de surface d'étanchéité prédéterminé ;
et **en ce que**
ledit motif de surface d'étanchéité est un motif hélicoïdal.

2. Système selon la revendication 1, dans lequel ladite surface d'étanchéité de chacune de la première enclume (14A) et de la seconde enclume (14B) est agencée dans un motif hélicoïdal incurvé.

3. Système selon la revendication 1 ou 2, dans lequel ladite surface d'étanchéité (26B) de chacune de la première enclume (14A) et de la seconde enclume (14B) inclut une pluralité de zones d'étanchéité en élévation qui sont agencées selon un motif présentant un angle (144) par rapport à l'enclume (14A, 14B).

4. Système selon l'une quelconque revendication 1 à 3, dans lequel un angle d'enclume et d'avertisseur (146) par rapport à la bande en défilement (12), conjointement avec le motif hélicoïdal de la surface d'étanchéité (26B) est configuré pour produire un collage qui est perpendiculaire à une direction de machine.

5. Système selon l'une quelconque revendication 1 à 4, dans lequel l'enclume (14A, 14B) présente une orientation inclinée.

6. Produit d'hygiène jetable produit avec un système de soudage (10) selon l'une quelconque revendication 1 à 5.
